# EUROPEAN PATENT SPECIFICATION

(11) **EP 2 101 806 B1**
(45) Date of publication and mention of the grant of the patent: **01.05.2013**
(21) Application number: 07847068.9
(22) Date of filing: 14.12.2007
(51) Int. Cl.: A61K 38/12, A61K 31/00, A61P 25/16

(54) **TREATMENT OF PARKINSON'S DISEASE OR PARKINSONIAN DISORDERS USING AGENTS THAT DECREASE THE ACTIVITY OF THE MELANOCORTIN 4 RECEPTOR**
BEHANDLUNG VON PARKINSON-KRANKHEIT ODER PARKINSON-ERKRANKUNGEN MIT MITTELN ZUR VERRINGERUNG DER WIRKUNG DES MELANOCORTIN-4-REZEPTORS
TRAITEMENT DE LA MALADIE DE PARKINSON OU DE TROUBLES PARKINSONIENS À L'AIDE D'AGENTS QUI RÉDUISENT L'ACTIVITÉ DU RÉCEPTEUR DE LA MÉLANOCORTINE 4

(30) Priority: 14.12.2006 US 875140 P
(43) Date of publication of application: 23.09.2009
(73) Proprietor: NeuroNova AB, 114 33 Stockholm (SE)
(72) Inventor: LINDQUIST, Per, 18250 Danderyd (SE); BERTILSSON, Goran, 137 41 Vasterhaninge (SE); MERCER, Alex, 122 63 Enskede (SE); PATRONE, Cesare, 112 67 Stockholm (SE); WIKSTROM, Lilian, 163 54 Spanga (SE); ZACHRISSON, Olof, 163 54 Spanga (SE)
(74) Representative: Mintz Levin Cohn Ferris Glovsky and Popeo LLP
(86) International application number: PCT/EP2007/010994
(87) International publication number: WO 2008/071438

(56) References cited:
- WO-A-03/040117
- WO-A-2004/045592
- WO-A-2005/081619
- WO-A-2005/103689
- GERAERTS MARTINE ET AL: "Concise review: therapeutic strategies for Parkinson disease based on the modulation of adult neurogenesis." STEM CELLS (DAYTON, OHIO) FEB 2007, vol. 25, no. 2, 2 November 2006 (2006-11-02), pages 263-270, XP002495311 ISSN: 1066-5099

## Description

### BACKGROUND

For several years, it has been established that neural stem cells exist in the adult mammalian brain. The first suggestions that new neurons were generated in the adult mammalian brain came from studies performed in the 1960s [1,2]. However, it took another three decades and refined technical procedures, to overthrow the theory that neurogenesis within the mammalian central nervous system (CNS) was restricted to embryogenesis and the perinatal period (for review see [3], [4]). Treatment of neural disease and injury traditionally focused on keeping existing neurons alive, but possibilities now exist for exploiting neurogenesis for therapeutic treatments of neurological disorders and diseases.

The source of new neurons is adult neural stem cells (NSC), located, e.g., within the ependymal layer and/or subventricular zone (SVZ) lining the lateral ventricle [5], [6] and in the dentate gyrus of hippocampus formation [7]. Other studies reveal the potential for several additional locations of NSC within the adult CNS [8]. In addition, there are indications that stem cells that can give rise to neural cells may come from outside the CNS. See, for example, Mezey, E. et al., "Turning Blood into Brain Cells Bearing Neuronal Antigens Generated in vivo from Bone Marrow." Science, Vol. 290, December 1, 2000, pp. 1779-1782, and Brazelton, T.R. et. al., "From Marrow to Brain: Expression of Neuronal Phenotypes in Adult Mice from Adult Bone Marrow-Derived Cells." Science, Vol. 290, December 1, 2000, pp. 1775-1779. Asymmetric division of NSCs maintain their number while generating a population of rapidly dividing precursor, or progenitor cells [6]. The progenitors respond to a range of cues that dictate the extent of their proliferation, survival and their fate, both in terms of cell type they differentiate into and the position they ultimately take up in the brain.

The NSC of the ventricular system in the adult brain are likely counterparts of the embryonic ventricular zone stem cells lining the neural tube whose progeny migrate away to form the CNS as differentiated neurons and glia. NSC persist in the adult lateral ventricle wall (LVW), generating neuronal progenitors that migrate down the rostral migratory stream to the olfactory bulb, where they differentiate into granule cells and periglomerular neurons [9]. Substantial neuronal death occurs in the olfactory bulb generating a need for continuous replacement of lost neurons, a need satisfied by the migrating progenitors derived from the LVW [10]. Further to this ongoing repopulation of olfactory bulb neurons, there are strong indications that lost neurons from other brain regions can be replaced by progenitors from the LVW that differentiate into the lost neuron phenotype complete with appropriate neuronal projections and synapses with the correct target cell type [11]; [12].

*In vitro* cultivation techniques have been established to identify the external signals involved in the regulation of NSC proliferation and differentiation [6], [13]. The mitogens EGF and basic FGF allow neural progenitors, isolated from the ventricle wall and hippocampus, to be greatly expanded in culture [14], [13]. The dividing progenitors remain in an undifferentiated state growing into large balls of cells known as neurospheres. Withdrawal of the mitogens combined with addition of serum induces differentiation of the progenitors into the three cell lineages of the brain, neurons, astrocytes, and oligodendrocytes [5, 6]. Application of specific growth factors can distort the proportions of each cell type in one-way or the other. For example, CNTF (Ciliary neurotrophic factor) acts to direct the neural progenitors to an astrocytic fate [15, 16], while the thyroid hormone, triiodothyronine (T3) has been shown to promote oligodendrocyte differentiation [16]. Enhancement of neuronal differentiation of neural progenitors by PDGF has also been documented [16]; [17].

The ability to expand neural progenitors and then manipulate their cell fate has enormous implications for treatment of neurological diseases in which specific cell types are lost (e.g. CNS degenerative disorders).

Examples of diseases characterized by neuronal cell loss are e.g. Parkinson's disease (PD), Huntington's disease (HD), Alzheimer's disease (AD) and amyotrophic lateral sclerosis (ALS), Lewy Body Diseases, multi-infarct Dementia, Pick's Disease, Creutzfeldt-Jakob Disease, frontal lobe degeneration (FLD, also called frontotemporal dementia or non-specific frontal lobe dementia), Corticobasal degeneration (CBD), multiple system atrophy (MSA), striatonigral degeneration (SND), progressive supranuclear palsy, Friedrich's ataxia, olivopontocerebe/lar atrophy (OPCA) and other CNS degenerative disease, stroke, brain trauma, epilepsia/LW and schizophrenia.

Examples of diseases characterized by glial cell loss include Charcot-Marie-Tooth disease, Guillain-Barre disease, multiple sclerosis, progressive multifocal leukoencephalopathy, acute disseminated encephalomyelitis (ADEM), HIV Encephalitis, central pontine myelinolysis, adrenoleukodystrophy, Krabbe's globoid cell, and metachromatic leukodystrophy, Alexander's disease, Canavan disease, Cockayne's syndrome, and Pelizaeus-Merzbacher's disease. Other disorders resulting in loss of glial cells include excessive radiation, side effects of chemotherapeutic agents (e.g., methotrexate, cis-platin, cytosine arabinoside (ARA-C), carmustine (BCNU), and thiotepa), and side effects with immunosuppressant therapy (e.g., cyclosporin A)

Furthermore, there are additional diseases such as multiple sclerosis (MS) which can be treated by immunosuppressive effects of undifferentiated neural stem/progenitors. See, e.g., Pluchino S, et al [18]. MS is characterized by loss of oligodendrocytes rather than neurons, by presumably autoimmune causes.

Progenitor cells may be expanded *in vitro* and subsequently transplanted to the patient. For the purposes of this disclosure, "patient" and "subject" have the same meaning. Previous transplantation treatments for PD patients have used fetal tissue taken from the ventral midbrain at a time when substantia nigral dopaminergic neurons are undergoing terminal differentiation [19]. The cells were grafted onto the striatum where they form synaptic contacts with host striatal neurons, their normal synaptic target, restoring dopamine turnover and release to normal levels with significant functional benefits to the patient [19] (for review, [20]). A much preferred alternative to transplantation is the strategy of stimulating the proliferation and differentiation of the endogenous progenitors *in vivo* using a pharmaceutical drug (i.e. growth factor, peptide or low molecular weight compound), administered directly to the brain or peripherally. Using this strategy, several problems associated with transplantation can be circumvented, such as lack of donor tissue and ethical questions raised using fetal tissue.

Intraventricular infusion to rat brain of EGF and basic FGF has been shown to proliferate the ventricle wall cell population. In the case of EGF, extensive migration of progenitors into the neighboring striatal parenchyma has been demonstrated [21, 22]. Differentiation of the progenitors was predominantly into a glial lineage, and the generation of neurons was reduced [21]. It has been shown that intraventricular infusion of BDNF in adult rats promotes an increase in the number of newly generated neurons in the olfactory bulb and rostral migratory stream, and in parenchymal structures, including the striatum, septum, thalamus and hypothalamus [23]. These studies demonstrate that the proliferation of progenitors within the SVZ of the LVW can be stimulated and that their lineage can be manipulated to produce neuronal and glial fates.

A number of growth factors, such as EGF and bFGF, have been identified as powerful mitogens of neural stem cells. Infusion into the lateral ventricle stimulates *in vivo* neurogenesis in normal mammals and critically in ischemic rats significantly augments injury upregulated neurogenesis, conferring functional gains. Vascular endothelial growth factor (VEGF) is another growth factor that promotes neurogenesis. Brain-derived trophic factor (BDNF) has also been shown to stimulate new neuron production in the striatum following intracerebroventricular infusion [23]. As does EGF, transforming growth factor alpha (TGF-alpha), functions through the EGF receptor stimulating neural stem cell proliferation. The effects of TGF-alpha have been studied in the rat 6-hydroxydopamine model of PD with fascinating results [24]. The 6-hydroxy-dopamine is one of the most widely accepted "classical" toxin-induced Parkinson's Disease models. The effect of the neurotoxin mimic the critical symptom related cellular loss seen in Parkinson's disease by destroying dopaminergic neurons in the substantia nigra. Infusion of TGF-alpha into the lesioned striatum (loss of SNpc dopaminergic neurons projecting axons to the striatum) induced rapid proliferation of subventricular zone stem cells followed by migration of a ridge of neuronal and glial progenitors directed towards the infusion / lesion site. Intriguingly, only in lesioned animals was this apparent. Subsequently, increasing numbers of differentiated neurons were observed in the striatum, a significant number of which stained positively for the dopaminergic neuron markers, tyrosine hydroxylase and dopamine transporter. In behavioral experiments, significant reductions of apomorphine-induced rotations were observed in the TGF-alpha treated animals. These results show that neural stem cells can be stimulated to produce new functional neurons in the striatum in a rodent PD model that reduce Parkinsonian symptoms. In this experiment, the neuronal replacement was not direct, in other words, the lost SNpc neurons were replaced by dopaminergic neurons, not in the SNpc, but in the striatum. Beneficial behavioral effects were presumably through the recovery of lost striatal dopaminergic inhibitory tone, normally provided by the SNpc dopaminergic neurons, but now released by the new striatal dopaminergic neurons.

In general, current Parkinson's disease drug therapies have involved drugs with temporary and short lasting effects which do not continue beyond the cessation of the drug therapy. Currently, the number of factors known to affect neurogenesis *in vivo* is small and their actions often, but not always, include the triggering of undesirable side effects. There is a long felt need for factors that can selectively stimulate neural stem cell activity, proliferation of neural progenitors, and differentiation of progenitors into the desired neuronal cell types. New methods are needed for stimulating *in vivo* neurogenesis (and/or neogenesis) and culturing cells for transplantation therapy.

It should be noted that to replace the neurons lost in neurodegenerative diseases, it could be beneficial if a subset of the progenitors adapt to a glial fate, rather than a neuronal fate. In addition to positive effects on the neurogenesis (and/or neogenesis) process itself and the resulting neurons, newborn glia cells can provide trophic support and neuroprotection to pre-existing neurons. This may be through the secretion of growth factors such as BDNF, GDNF or PDGF, or neuropeptides such as PACAP, VIP, GLP1, or chemokines and cytokines, which confer these effects on the neurons. Beneficial effects on the neuronal network from the secretion of these factors from new glia cells may also come in the form of increased neuronal arborisation, synaptogenesis or regulation of transporters and receptors critical to the efficiency of synaptic transmission.

Brain inflammation plays an important role in the pathogenesis of chronic neurodegenerative diseases, such as Alzheimer's and Parkinson's disease. Neurodegeneration caused by inflammation involves activation of the brain's resident immune cells, the microglia, which produce a large number of pro-inflammatory neurotoxic factors. Also, acute brain insult, such as stroke and seizures are linked to inflammation which contributes to the propagation of the neuropathological events. The formation of new glia cells secreting factors into the brain parenchyma may enable the brain to modulate the immune response, thus reducing the adverse effects of inflammation and promoting the beneficial effects. Immature neural progenitors have immunomodulatory properties (See reference in earlier comment).

Pharmaceutical drugs could promote neural stem cell progeny to produce factors or processes beneficial for the neuronal networks. This could be the case for newborn glia or for that matter pre-existing glia/cells that are induced to produce and/or secrete trophic, neuroprotective, immune modulating factors or newborn neurons or pre-existing neurons that are stimulated to improve their functionality.

The result of all of these mechanisms of action we propose would be of benefit to patients with disease or injury of the nervous system.

### Melanocortin Receptors

Five subtypes of melanocortin receptors, MC1-5R, that belong to the class A GPCR superfamily have been identified and cloned [25]. These receptors are involved in a multitude of functions. The MC1R regulates skin pigmentation and the immune system. The MC2R (ACTH receptor) controls steroid production. The MC3R might be involved in regulation of central sexual behavior, the MC5R has a role for regulating exocrine gland secretion whereas MC4R, mainly controls feeding behavior [26]. Agonists to MC4R reduce food intake whereas antagonists increase food intake. Other functions for the MC4R involve erectile activity, nociception, anxiety/depression and regulation of the Hypothalamic-Pituitary-Adrenal axis (reviewed in [27]).

The MC4R is expressed primarily in the brain as assessed by Northern blot [28]. Using *in situ* hybridization technique, it has been shown that MC4R is widely distributed in the brain, at multiple sites including the cortex, thalamus, hypothalamus, and brainstem [29], [30]. Furthermore, moderate to extensive labeling is also seen in amygdala, hippocampus and enthorhinal cortex [30].

The natural ligands (agonists) for the melanocortin receptors consist of the melanocyte-stimulating hormones (MSH) alpha, beta, and gamma, and the adrenocorticotropin ACTH. All melanocortin receptors are activated by ACTH, whereas all melanocortin receptors except MC2R are activated by MSH as shown in the following chart:

| **Melanocortin Receptor** | **Ligands Known to Activate MCR** |
|---|---|
| MC1R | ACTH, alpha, beta, and gamma-MSH, Agouti |
| MC2R | ACTH |
| MC3R | ACTH, alpha, beta, and gamma-MSH, AGRP |
| MC4R | ACTH, alpha, beta, and gamma-MSH, Agouti, AGRP |
| MC5R | ACTH, alpha, beta, and gamma-MSH |

In addition, two endogenous antagonists, agouti-protein and agouti-related protein (AGRP), have been identified [31]. Agouti is expressed in human adipose tissue, testis, ovary, heart, and at lower levels in foreskin, kidney, and liver. AGRP was cloned based on its homology to agouti (25% identity with human agouti). AGRP has a very distinct distribution in the central nervous system, being expressed in neuronal cell bodies of posterior hypothalamus in close vicinity to proopiomelanocortin (POMC)-expressing neurons. Agouti is a competitive antagonist at MC1R and MC4R, but does not bind to MC3R and MC5R. In contrast, AGRP binds to and antagonizes MC3R and MC4R, and acts as an inverse agonist at MC4R (reviewed in [27]). While current research of the melanocortin system has focused on MC4R agonists for the possible treatment of obesity, MC4R antagonists have been used for the possible treatment of cachexia (physical wasting with loss of weight and muscle mass caused by disease). It has also been suggested that blockage of the MC4R could be a useful way of alleviating symptoms of anxiety pain and addiction to drugs of abuse (reviewed in [27]). Use of agents that decrease the activity of MC4R to treat neurodegenerative diseases or injuries of the central nervous system has not been described previously.

Modulating neogenesis may involve changing (increasing or decreasing) many characteristics of a cell or its progeny. These characteristics include, at least, proliferation, survival, differentiation, de-differentiation, migration and the production or secretion of an agent (such as a protein). Where modulating neogenesis involved changing the expression or secretion of an agent, the agent may be a trophic factor, an immunomodulatory factor, a neuroprotective factor, a neuronal arborisation factor, a synaptogenesis promoting factor, or a synaptic transmission promoting factor of the neural cell or its progeny. In addition, modulating CNS neogenesis may involve modulating the immunomodulatory effects of the neural cell or its progeny.

Any one of the methods disclosed herein is applicable to CNS cells of any mammalian origin. Thus, the methods are applicable to CNS cells from humans, dog, cat, mice, rabbit, cow, pig, horse, goat, sheep and other commercially valuable mammals. The CNS cells may be of any stage in the life of the mammal which contains CNS cells including adult CNS cells, juvenile CNS cells, prepubescent CNS cells and newborn CNS cells. Examples of these cells include, at least, adult neural stem cell, and a non-embryonic stem cell.

MC4R activity decreasing agents used as agents in the methods of the invention are selected from the group consisting of HSO14, HS028, compound 10, compound Pontillo14c, compound Xi 14a, Xi 14b, Xi14c, Xi14d, Xi14e, Xi14f, Xi14g, Xi14h, Xi14i, Xi14j, SHU9119, HS024, Compound 10d, Compound 18v, Compound 13b-2, Compound Tran12e, and analogs and low molecular weight analogs and combinations thereof. Use of Agouti (1-40) amide and Agouti (87-132) is also disclosed.

The methods of the invention may further include an optional step of contacting the cell with one or more growth factors. These growth factors may be, for example, EGF, PDGF, FGF, TGF-β, TGF-α, Epo, IGF-I, IGF-II, IL-I , IL-2, IL-6, IL-8, TNF-α, TNF-β, IFN- β, IFN-γ, CSF, VEGF or a combination thereof. These growth factors may be applied before, during, or after the application of the agent(s) decreasing the activity of MC4R.

The disorders that can be treated by the method of the invention include Parkinson's disease or Parkinsonian disorders.

Treatment of those disorders characterized by an abnormal reduction of neurons, or an abnormal reduction of glial cells in the subject, such as Huntington's disease, Alzheimer's disease, amyotrophic lateral sclerosis, spinal ischemia, ischemic stroke, spinal cord injury, cancer-related brain injury, and cancer-related spinal cord injury, Shy-Drager syndrome, progressive supranuclear palsy stroke, cerebral infarction, multi-infarct dementia, geriatric dementia, Lewy Body Diseases, Pick's Disease, Creutzfeldt- Jakob Disease, frontal lobe degeneration, Corticobasal degeneration (CBD), multiple system atrophy (MSA), striatonigral degeneration (SND), progressive supranuclear palsy, Friedrich's ataxia, and olivopontocerebe/laratrophy (OPCA), multiple sclerosis and epilepsy is also disclosed.

We also disclose treatment of disorders including neurodegenerative disorders, neural stem cell disorders, neural progenitor disorders, ischemic disorders, neurological traumas and injuries, affective disorders, neuropsychiatric disorders, degenerative diseases of the retina, retinal injury and trauma, learning and memory disorders, ataxia associated with neurodegeneration, equine degenerative myelo-encephalopathy, cerebellar abiotrophy, equine motor neuron disease; grass sickness (equine dysautonomia), postanaesthetic myelomalacia, and equine leuko-encephalomalacia.

As an optional step, the administration may include the administered one or more agents before, after, or during administration of the agent decreasing the activity of MC4R. The agent may be one of the growth factors listed above. In addition, the agent may be antidepressants, anti-anxiety agents, anti-psychotic agents, anti-epilepsy agents, anti-Alzheimer's agents, anti-Parkinson's agents, MAO inhibitors, serotonin-uptake blockers, noradrenaline uptake blockers, dopamine uptake blockers, dopamine agonists, L-DOPA, tranquilizers, sedatives, and lithium.

Compositions may be administered using any administration method. These methods include systemic administration and central administration. Methods of systemic administration include oral, subcutaneous, intracutaneous, intravenous, intraarterial intraperitoneal, intramuscular, buccal, mucosal, nasal, pulmonary, and rectal administration. In a preferred embodiment, the compositions of the invention are administrated by a nasal spray or nasal suppository. For example, nasal administration may be performed using a dry powder inhaler or aqueous-based inhaler. In a preferred embodiment, the use of a MC4R activity decreasing agent in methods of the invention involve administration to the CNS of the subject (central administration), such as intraventricular, intraparenchymal, intrathecal and intracranial administration. This may be performed, for example, by injection or infusion.

We also disclose a method of inducing CNS neogenesis in a patient exhibiting at least one symptom of a central nervous system disorder. The method includes the step of administering an agent decreasing the activity of MC4R to said patient in a sufficient amount so that the agent induces CNS neogenesis in the patient. CNS neogenesis comprises inducing conversion of a neural cell into an oligodendroglia cell. The conversion further may comprise the steps of (a) converting the neural cell into a stem cell (e.g., unipotent, oligopotent, or pluripotent stem cell), and (b) converting the stem cell into an oligodendroglia cell. Furthermore, this conversion step may be performed *in vitro.* That is, the neural cell may be removed from a patient and treated with an agent decreasing the activity of MC4R to become converted to an oligodendroglia cell. Then the oligodendroglia cell may be reimplanted into a patient.

The determination of an effective amount of an agent decreasing the activity of MC4R, such as a MC4R antagonist to be administered is within the skill of one of ordinary skill in the art and will be routine to those persons skilled in the art. The amount of MC4R activity decreasing agent to be administered will depend upon the exact size and condition of the patient, but will be at least 0.1 ng/kg/day, at least 1 ng/kglday, at least 5 ng/kg/day, at least 20 ng/kg/day, at least 100 ng/kg/day, at least 0.5 ug/kg/day, at least 2 ug/kg/day, at least 5 ug/kg/day, at least 50 ug/kg/day, at least 500 ug/kg/day, at least 1 mg/kg/day, at least 5 mg/kg/day, at least 10 mg/kg/day, or 1-100 mg/kg/day in a volume of 0.001 to 10 ml. In another method of dosage, the agent may be administered so that a target tissue achieves an agent concentration of 0.0001 nM to 500 nM, 0.001 nM to 500 nM, 0.01 nM to 500 nM, 0.1 nM to 500 nM, 0.1 nM to 100 nM, or at least 1 nM, at least 50 nM, at least 100 nM, or at least 500 nM. Preferred dosages include systemic administration of at least 10 mg twice a day or at least 25 mg twice a day; systemic administration of at least 0.04 mg/kg/day, at least 0.08 mg/kg/day, at least 0.24 mg/kg/day, at least 36 mg/kg/day, or at least 48 mg/kg/day; systemic administration of at least 22 mcg twice a day or 44 mcg twice a day; or systemic administration of at least 3-10 mg/kg once a week. Particularly preferred dosage ranges are 0.04 mg/kg to 4 mg/kg and 0.05 mg/kg to 5 mg/kg. These dosages may be increased 10x, 100x or 1000x in transdermal or topical applications.

For any of the methods of the disclosure, administration may be by a number of routes depending on the nature of the results to be achieved. Administration may be oral, nasal, topical or by injection or infusion. The routes of parenteral administration may be by injection or infusion includes intravenous (IV), intraperitoneal (IP), subcutaneous (SC), intramuscular or intramedullary (i.e., intrathecal) injection and intracerebralventricular (ICV). The agents to be administered may be liquid, solid, pill, suppository, nasal spray or any known format for delivery to a patient - which can be any mammal including, of course, humans.

Another embodiment of the invention is directed to use of a MC4R activity decreasing a method for alleviating a symptom of a degenerative CNS disorder as defined in claim 1, wherein said method further comprises monitoring an indicia of neogenesis in the subject by a non-invasive method to determine if the doses are sufficient, and increasing the dosage (i.e., the doses) if the indicia of neogenesis is below a minimum threshold or decreasing the dosage if the indicia of neogenesis is above a maximum threshold.

The monitoring step may be performed by a number of methods. We have found that one reliable and predictable indicia of agent effectiveness is weight gain. Moreover, we have found that weight gain is particularly reliable in subjects with a degree of CNS disorder - such as Parkinson's disease or a chemically induced model of Parkinson's disease By measuring weight gain, a sufficient dose is determined as the minimum dose that causes an increased in body weight of the subject above a set threshold compared to a second subject with a similar CNS disorder symptom but not administered said composition. The threshold may be, for example, a body weight gain of 1%, 2%, 3%, 4%, 5%, 6%, 7%, 8%, 9%, 10%, 12%, 15%, 20%, 40% or 50%. An increase in body weight is most easily measured by providing a subject with the same food and drink freely so that any weight gain is not due to a change in the quality or quantity of food available. Naturally, under these controlled conditions, the patient should not be administered a appetite or weight affecting agents except for the agent being tested - the MC4R activity decreasing agent.

The weight gain or loss can thus be determined by comparison with a second subject with the same degree of CNS disorder and symptoms but which is not administered the agents of the invention. Another useful comparison would to compare the weight of the subject before treatment and the weight of the subject during treatment. That is, the original subject and the second subject may be the same subject at a different time.

Another noninvasive method for monitoring neogenesis is to monitor a neural stem and progenitor cells levels in a CNS of the subject by proton nuclear magnetic resonance. In a publication [32], the authors describe a metabolic biomarker for the detection and quantification of neural progenitor cells (NPCs) in the human brain in vivo. The authors used proton nuclear magnetic resonance spectroscopy (¹H-MRS) to identify and characterize a biomarker in which NPCs are enriched and demonstrated its use as a reference for monitoring neurogenesis. To detect low concentrations of NPCs in vivo, the authors developed a signal processing method that enabled the use of magnetic resonance spectroscopy for the analysis of the NPC biomarker in both the rodent brain and the hippocampus of live humans. The exact molecular nature of the biomarker was not yet established but there were strong indications that the "1.28-ppm spectral peak"-biomarker described by the authors is a complex mixture of saturated and/or monounsaturated fatty acids and related compounds. These findings thus open the possibility of investigating the role of NPCs and neurogenesis in a wide variety of human brain disorders in a non-invasive manner.

As stated above, the methods of the invention are particularly effective for subjects with a degree of CNS disorder. For example, the subject may have a decrease of dopaminergic neurons compared to a healthy subject of at least 30%, at least 50%, at least 75%, at least 80%, at least 85%, at least 90%, at least 95% and at least 99%.

In another embodiment of the invention, the use in treating a CNS disorder comprises administering to the subject the MC4R activity decreasing agent for a period of time until the subject displays a desired reduction in degenerative CNS symptoms, and wherein the subject shows a continued reduction in symptoms for a period of at least two weeks after the administration of said MC4R activity decreasing agent is stopped. The degenerative CNS disorder is Parkinson's disease and the degenerative CNS symptoms is a Parkinson's disease symptom. The Parkinson's disease symptom may be a decrease in dopaminergic neurons in the CNS. Also disclosed herein are other degenerative CNS diseases, for example, Huntington's disease, Alzheimer's disease, amyotrophic lateral sclerosis, Lewy Body Diseases, multi-infarct Dementia, Pick's Disease, Creutzfeldt-Jakob Disease, frontal lobe degeneration, Corticobasal degeneration, multiple system atrophy, striatonigral degeneration, progressive supranuclear palsy, Friedrich's ataxia, olivopontocerebellar atrophy, stroke, brain trauma, epilepsia, schizophrenia, Charcot-Marie-Tooth disease, Guillain-Barre disease, multiple sclerosis, progressive multifocal leukoencephalopathy, acute disseminated encephalomyelitis (ADEM), HIV Encephalitis, central pontine myelinolysis, adrenoleukodystrophy, Krabbe's globoid cell, and metachromatic leukodystrophy, Alexander's disease, Canavan disease, Cockayne's syndrome, and Pelizaeus-Merzbacher's disease, excessive radiation, and side effects of chemotherapeutic agents, and side effects with immunosuppressant therapy. This method has many utilities in treating degenerative CNS disorders. For example, this method may be used to increase the proliferation of adult neural stem cells in the lateral ventricular wall. The MC4R antagonist activity decreasing agent may be administered at a dosage of between 0.1 ng/kg/day to 10 ng/kg/day, between 1 to 100 ng/kg/day, or between 10 and 1000 ng/kg/day. Administration may involve any method recited in this disclosure including the direct administration by injection or infusion of the agent to the CNS of the subject. An alternative method of measuring dosage is to provide a dosage so that the subject achieves a tissue concentration of 0.1 nM to 500 nM.

### BRIEF DESCRIPTION OF THE FIGURES

- Figure 1: depicts A) the MC4R antagonists HS014 or HS028 within 1-100nM concentration increase ATP levels in suspension cultures of adult neural stem cells; and B) HS014 within 1-100nM concentration increase BrdU incorporation in adherent cultures of adult neural stem cells.
- Figure 2: depicts A) Effect of HS014 on the proliferation in the SVZ. Administered through osmotic minipumps into the right lateral ventricle of rats over a two weeks period; B) Effect of HS014 on body weight. Administered through osmotic minipumps the right lateral ventricle of rats over a two weeks period. ** P < 0.01
- Figure 3: depicts structures of some MC4R antagonists.
- Figure 4: depicts the effect of HS014 on net ipsiversive rotations induced by amphetamine in the 6-OHDA-lesioned rat model of Parkinson's disease. **P < 0.01 vs. Baseline; one-way repeated measures ANOVA followed by Dunnett's test. # P < 0.05 vs. respective vehicle; unpaired t-test.
- Figure 5: depicts the effect of HS014 on body weight. Time on the x-axis is in weeks. The period of administration is indicated by the shaded box. The average body weights of the treated group is represented by boxes, while the placebo group is presented by diamonds.

### DETAILED DESCRIPTION OF THE INVENTION AND DISCLOSURE

### Definitions

A stem cell refers to any neural stem cell including unipotent, oligopotent or multipotent (pluripotent) stem cells. Unipotent, oligopotent or multipotent (pluripotent) stem cells are defined as cells that can differentiate into one, or many cell types. Naturally, stem cells include progenitor cells which can differentiate into neural cells. Stem cells also include neogenic precursors (NGP) which is a designation of any precursors of neural cells including neural stem cells, non-lineage restricted neural progenitor cells and lineage restricted neural precursor cells. Any precursor cells of non-CNS origin capable of migrating into the CNS are also included in NGP cells. The term "stem cell" and "neural stem cell" is understood to include, at least, adult neural stem cells, juvenile neural stem cells, non-newborn neural stem cells, and non-embryonic neural stem cells.

A neural cell (as opposed to a neuronal cell) is any cell in the nervous system which includes nerve cells (neurons), glial cells (glia) and any cell that give rise to new cells in the brain by differentiation or division. A neural stem cell is a cell that can produce a neural cell as a progeny. In this case, progeny may be a direct progeny such as a daughter cell or a more distant progeny such as a granddaughter cell, great granddaughter cell, and so on. The definition of a "neural cell" include, at least, all the major mature CNS cell types, such as, for example neurons, astroglia, NG2 positive glia, and oligodendroglia cells as well as stem cells that give rise to cells of a neural lineage.

An antagonist of a receptor means a molecule or a group of molecules able to bind to the receptor and block the binding of a ligand, including a natural ligand, to the receptor. For example, an "antagonist" of receptor can be a ligand which competitively binds to the receptor at the same site as the natural ligand, but does not activate an intracellular response initiated by an active form of a receptor, and thereby inhibits the intracellular response induced by the natural ligand. An antagonist may, for example, reduce a cellular response of a cell to the compound by at least 10%, preferably 15-25%, more preferably 25-50% and most preferably, 50-100%, as compared to the intracellular response in the presence of the natural ligand in the absence of said antagonist. An antagonist analog is a compound that can induce a cellular response of an antagonist when the antagonist analog is contacted to a cell, a cell culture, or a tissue containing the cell. The analog may work directly, by binding a receptor on a target cell. Alternatively, the analog may act indirectly, by binding or influencing a neighboring cell and affecting the target cell to achieve a similar outcome as if an antagonist is applied. Antagonist analogs may work by many mechanisms. For example, an antagonist analog may block intracellular signaling such that even though a ligand is bound to the receptor on the cell, the cell does not react to the bound ligand in the presence of the antagonist analog.

Examples of MC4R activity decreasing agents which may be used in the methods of the invention are here exemplified by the two cyclic peptides HS014 and HS028. HS014 has the amino acid sequence of:
[acetyl group]-Cys-Glu-His-(D-2-Nal)-Arg-Trp-Gly-Cys-Pro-Pro-Lys-Asp-[amide group] (SEQ ID NO:3, CAS Number 207678-81-7, MDL number MFCD02179654)

This listing is written from the N terminus to the C terminus and as shown above, there is an acetyl group at the N terminus and an amide group at the C terminus. Unless defined otherwise, all peptides and proteins are listed in this disclosure is in the conventional form starting from the amino terminus to the carboxyl terminus (i.e., amino terminus - Xxx-Xxx-carboxyl terminus). The amino acids that make up the ring closure are underlined and Nal refers to beta-naphthylalanine. See, Kask et al. [33]. HS028 has the amino acid sequence
[acetyl group]-Cys-Glu-His-(diCl-D-Phe)-Arg-Trp-Gly-Cys-Pro-Pro-Lys-Asp-[amide group] (SEQ ID NO:4,)
where "diCl-D-Phe" refers to dichloro-D-phenylalanine. These two antagonists of MC4R have been used as a tool in cachexia/anorexia research. In one investigation, HS014 was injected i.c.v. either by twice-daily injections (2 x 1 nanomoles) for 6 days or by continuous infusion with osmotic minipumps (0.16 nanomoles per hour) for 2 weeks. The results showed a considerable increase in food intake and body weight after both of the treatments without any signs of tachyphylaxis [34]. Other useful MC4R antagonists are as follows:

Compound 14c (referred to herein as Pontillo14c):

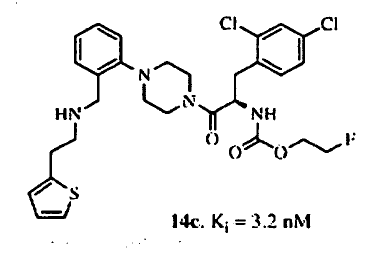

Pontillo14c is described in Pontillo, J. et al.[35]. A potent and selective nonpeptide antagonist of the melanocortin-4 receptor induces food intake in satiated mice, Bioorganic & Medicinal Chemistry Letters, Volume 15, Issue 10, 16 May 2005, Pages 2541-2546.

### 4-{(2R)-[3-Aminopropionylamido]-3-(2,4-dichlorophenyl)propionyl}-1-{2-[(2-thienyl)ethylaminomethyl]phenyl}piperazine (preferred to herein as compound 10):

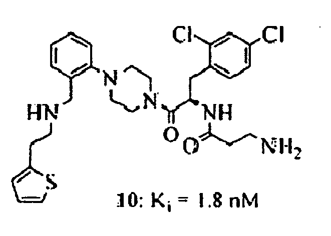

This compound is listed as compound 10 in Chen, C. et al. [36] 4-{(2R)-[3-Aminopropionylamido]-3-(2,4-dichlorophenyl)propionyl}-1-{2-[(2-thienyl) ethylaminomethyl]phenyl}piperazine as a potent and selective melanocortin-4 receptor antagonist-design, synthesis, and characterization.

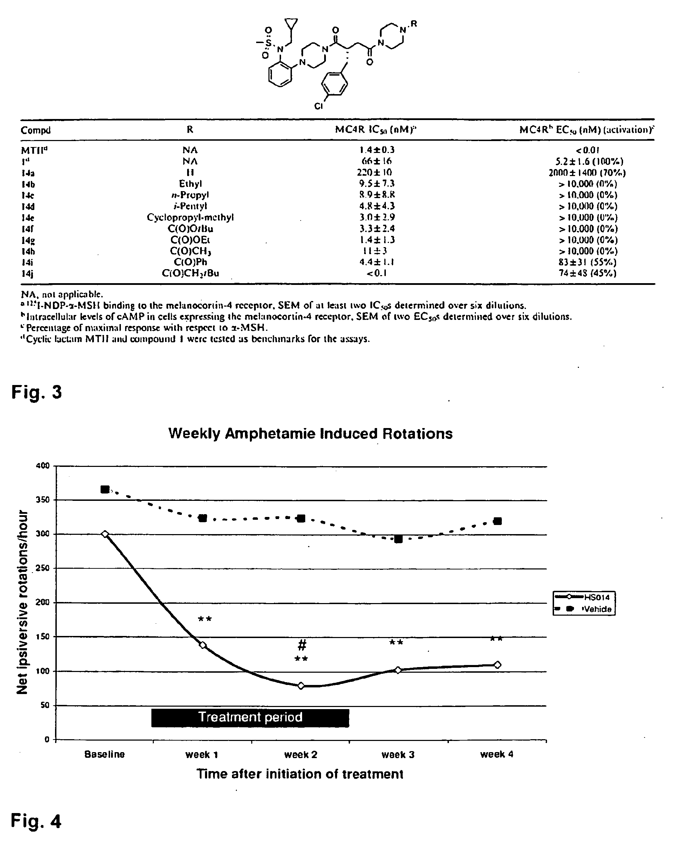

Compounds 14a to 14j (referred to herein as compounds Xi14a to Xi14j) of Figure 3 as described in Xi N., et al., Synthesis of novel melanocortin 4 receptor agonists and antagonists containing a succinamide core. [37]. (Note, while the compounds in Xi have the same name (i.e., compound 14) they are not related to Pontillo14c above.).

Other known compounds useful for the methods of the invention include S1HU9119, HS024, Compound 10d, Compound 18v, Compound 13b-2, Compound and Tran12e. A summary of the mentioned compounds, their properties and relevant references describing these compounds in detail is given in Table 1 below:

**Table 1: Summary table of selected compounds useful for the methods of the invention**

| ***Compound*** | ***Selectivity (over MC3)*** | ***Affinity for MC4R (Ki)*** | ***Scientific Reference(s)*** |
|---|---|---|---|
| HS014 | 10-fold | 3,2 nM | [33], [38] |
| HS028 | 80-fold | 0,95 nM | [39] |
| SHU9119 | equal | 0,36 nM | [38] |
| HS024 | 20-fold | 0,29 nM | [40] |
| Compound 10 | No potency i cAMP assay, Ki at MC3R=640 nM | 1,8 nM | [36] |
| Pontillo14c | 240-fold | 3,2 nM | [35] |
| Compound 10d | ? | ? | [41] |
| Compound 18v | | 0,5 nM | [42] |
| Compound 13b-2 | Ki at MC3R=1300 nM | 4,7 nM | [43] |
| Compound Tran12e | 1800 nM | 11 nM | [44] |
| Compounds Xi14a-j | Several | several | [37] |
| ML00253764 | | | [45] |
| MCL0042 | | | [46] |
| Agouti (1-40) amide | | | [31] |
| Agouti (87-132) | | | [31] |

The MC4R antagonists listed above are examples of compounds useful for the methods disclosed. Many other useful compounds are known within the art, see e.g. patents/patent applications EP1468999, EP1460070, PCT/EP2007/003115, PCT/EP2007/001595, PCT/EP2004/002907, PCT/EP2004/002896, PCT/EP2004/002908, PCT/EP2004/002909, PCT/US2002/032282, PCT/US2003/004455, PCT/US2003/ 014628, PCT/US2003/040931, PCT/US2004/035343, PCT/US2004/034951. PCT/US2002/023926, PCT/US2002/023616, and academic publications [47, 44, 48, 49, 50]

For any of the uses intreating a CNS disorder of the invention, the dosage for the MC4R antagonist may be (1) alone in a dosage range of 0.001 ng/kg/day to 500 µg/kg/day, preferably in a dosage range of 0.05 to 150 or up to 300 ng/kg/day, (2) in a combination permeability increasing factor, or (3) in combination with a locally or systemically co-administered agent. Dosage ranges of, for example, 1 to 10 ng/kg/day, 10 to 100 ng/kg/day, 20 ng/kg/day to 2000 ng/kg/day and from 100 ng/kg/day to 500 ng/kg/day have been found to be particularly effective for the methods of the invention. The administration may lead to tissue concentrations of the agent of about 0.0001 nM to 5000 nM. The administration may also lead to cerebrospinal fluid concentrations of about 0,001 nM to 10000 nM, preferably 0.1 to 1000 nM, more preferably 1 nM to 100 nM, even more preferably 3 nM to 30 nM and most preferably about 10 nM.

One method of selecting additional MC4R antagonist is to produce variants of an antagonist to see if it has the same effects. Methods of producing variants are known. For example, amino acid sequence variants of peptides can be synthesized or made through cloning and mutations. Such variants include, for example, deletions from, or insertions or substitutions of, residues within a peptide. Any combination of deletion, insertion, and substitution can be made to arrive at the final peptide/antagonist, provided that the final peptide/antagonist possesses the desired characteristics - which can be tested using the methods disclosed in the Example section. Still other variants may be made by introducing or moving sugar groups in an antagonist (e.g., glycosylation sites).

"An agent decreasing the activity of MC4R" can be an antagonist, an inverse agonist, an inhibitor, or an agent lowering the expression level of MC4R, as defined below. Any compound or agent that decreases the activity of MC4R is encompassed in the term. For the purposes of this disclosure, the words "compound" and "agent" have the same meaning.

Identifying compounds that are useful is known to one skilled in the art based on the disclosures presented here. Assay kits for measuring ligand binding to MC4R-receptor are commercially available (e.g Melanocortin MC4 ¹²⁵I - SPA GPCR kit cat: MRP0070 from GE Healthcare). Using such a kit, one skilled in the art can easily determine whether a given compound can bind to the MC4R-receptor. Methods for determining the effect a given compound has on the activity of MC4R-receptor are also well known in the art. Most commonly utilized methods involve introducing the receptor via recombinant DNA technology to a cell line, and measuring the effects test compounds have on cAMP production or receptor translocation. These and other known techniques are disclosed in public literature, e.g. Schiöth et al. [51] and international patent applications WO2005103715 and WO2005103689. Obtaining such measurements for a given compound are also commercially available as a service, such as from Cerep (Paris, France, www.cerep.com), catalog numbers 758-18a and 18b for MC4R agonist and antagonist effects, respectively.

Utilizing these known methods, one skilled in the art may determine whether a given compound that binds to the MC4R-receptor increases or decreases the activity of the receptor. When assaying activity of a compound in the absence of a known agonist, agonists produce the result of a dose-dependent increase in activity, whereas inverse agonists result in decreased activity. When assaying activity of a compound in the presence of a known MC4R-agonist such as α-MSH, antagonists and inhibitors result in a dose-dependent decrease in the activity of the receptor.

Reducing the number of available MC4R-receptors in a target cell is another way of achieving the desired effect of the invention, namely decreasing MC4R-activity. Agents known in the art to achieve this effect include an antisense polynucleotide, a ribozyme, and a small interfering RNA (siRNA), wherein said agent comprises a nucleic acid sequence complementary to, or engineered from, a naturally occurring polynucleotide sequence encoding a MC4R-polypeptide. A vector can be used to express said agent, such as a adenoviral, retroviral, adeno- associated viral, lentiviral, a herpes simplex viral or a sendaiviral vector.

A functional analog of a ligand is a compound which induces the same or a similar activity in the cell as the ligand when the analog is contacted with a cell - regardless of the mechanism of action of the analog.

A functional analog also comprises an "inverse agonist." As used herein, an "inverse agonist" of a compound refers to a ligand which decreases a constitutive activity of a cell surface receptor when it binds to a receptor. An inverse agonist may decrease the constitutive intracellular response mediated by a receptor by at least 2-fold, preferably 5-fold, more preferably 10-fold and most preferably 100-fold or more (i.e., 150-fold, 200-fold, 250-fold, 500-fold, 1000-fold, 10,000-fold etc), as compared to the intracellular response in the absence of inverse agonist and in the absence of any compound with an agonist activity.

An "inhibitor" compound is a molecule directed against the receptor or against a natural ligand for the receptor that decreases the binding of the ligand to the receptor by at least 10%, preferably 15-25%, more preferably 25-50% and most preferably, 50-100%, in the presence of the compound, as compared to the binding in the presence of the compound and in the absence of inhibitor. An "inhibitor" compound can decrease the intracellular response induced by an agonist by at least 10%, preferably 15-25%, more preferably 25-50% and most preferably, 50-100%. As an example, an inhibitor may bind a compound and prevent the compound from binding a receptor.

As used herein, "natural ligand" refers to a naturally occurring ligand, found in nature, which binds to a receptor. A "natural ligand" does not refer to an engineered ligand that is not found in nature and that is engineered to bind to a receptor.

### Methods for Inducing Neogenesis in Central Nervous System Tissue (CNS)

We disclose novel methods, compounds and compositions for modulating (increasing or decreasing) neogenesis in CNS tissue. The term "neogenesis" has the same meaning as "CNS neogenesis" in this disclosure and refers to the growth of new cells in the CNS. Central nervous system tissue is well known and includes, at least, the spinal cord, medulla, pons, midbrain, cerebellum, diencephalon, cerebral hemispheres and all the tissues in each of these major divisions (See, e.g., Chapter 17 of Kandel, E.R., et al. Principles of Neural Science, 4^{th} edition, McGraw Hill (New York) 2000).

The methods for inducing neogenesis refer to the development of additional neural cells and tissue in a postnatal mammal by any mechanism, including, at least, the following: (1) the growth (proliferation) (2) differentiation of new neural cells, (3) the migration of existing or new neural cells into a needed region, (4) the conversion of one neural cell type into a different neural cell type, (5) the conversion of a non-neural cell type (e.g., stem cell from non CNS tissue) into a neural cell type (6) preventing conversion of neural cells into non-neural cells in the CNS; (7) preventing natural or disease induced death of neural cells, (8) the activation of proliferation or differentiation through factors secreted or expressed by neural cells, (9) the inducement of in-migration of neural cells to a region which is in need of neogenesis, such as, for example, including NGP, neural cells and glial cells to migrate to the peripheral nervous system for the treatment (e.g. in MS). One form of CNS neogenesis would involve modulating the proliferation, differentiation, survival or migration of neural cells and neural stem cells.

In any of the methods disclosed herein, modulating neogenesis may also involve administering an agent decreasing the activity of MC4R to a subject and modulating neogenesis indirectly. Indirect methods of modulating neogenesis may involve, for example, causing a neural cell or a non-neural cell to express and/or secrete growth factors to support neogenesis. As another example, the agent decreasing the activity of MC4R may induce a glial cell to express and/or secrete growth factors to support the proliferation of neurons, or, the agent decreasing the activity of MC4R may induce a neuron to express and/or secrete a growth factor to support the growth of glial cells. As a further example, the agent decreasing the activity of MC4R may cause a non-neural cell to express and/or secrete a growth factor to support the growth or proliferation of the neural cell. It is understood that growth factors may mediate their function by expression on the cell surface of one cell, and, through cell-to-cell contact, induce neogenesis of a second cell. In this scenario, a growth factor does not have to be secreted to mediate its function. It is also understood that a cell can secrete and/or express growth factors for its own use or for the use similar type of cell and that such secretion or expression is also encompassed by the methods here described.

As an example of CNS neogenesis, the process of neogenesis with respect to specific cell type is discussed in the following paragraphs. With respect to stem cells (or any individual neural cell type), neogenesis may involve, for example, the proliferation of stem cells into more stem cells. In this case, neogenesis involves modulating the proliferative activity of NGP cells. Neogenesis may also involve modulating the differentiation pathway of stem cells into neural cells. Modulation may involve changing both the direction of differentiation and the number of progeny cells produced after the differentiation. In addition, NGP cells can differentiate into beneficial neural cell types, or elicit a beneficial effect as undifferentiated cells. Further to this example, neogenesis may also involve the inducement of neural cells or stem cells to migrate into a needed area of the CNS (e.g., brain) - by modulating the migration of NGP cells. As another example, neogenesis may modulate (e.g., promote and increase) the survival of NGP cells or their progeny.

Neogenesis may also occur by the conversion of a cell of one lineage (e.g. neurons, astroglia, or oligodendroglia) into a second different lineage. There are many mechanisms for the conversion of one cell lineage into another lineage. Each of these mechanisms is discussed below. In the most direct mechanism, cells of one lineage may convert into a second lineage directly. For example, a neuron can convert into an astroglia cell or an astroglia cell may convert into a neuron. Alternatively, the conversion may involve an intermediate cell type such as the conversion of a neuron into a stem cell (unipotent, oligopotent, or pluripotent), followed by the conversion of the stem cell into an oligodendroglia). In another mechanism, fully differentiated neural cells can de-differentiate back into NGP cells. The NGP cell may further differentiate into the original neural cell type or a new neural cell type. Neogenesis may also involve modulating the de-differentiation of neural cells.

Neogenesis may involve modulating a function of NGP cell in a patient in need of such modulation. The function that is modulated may include, at least, the following: (1) modulating the secretory activity of NGP cells which may secrete, for example, additional growth factors to support neogenesis or the growth and preservation of any neural cell, (2) modulating the immunosuppressive activity of NGP cells, (3) modulating the proliferative activity of NGP cells, (4) modulating the differentiation of NGP cells, (5) modulating the migration of NGP cells, (6) modulating the survival of NGP cells or their progeny, and (7) modulating the de-differentiation of neural cells into NGP cells.

While examples of neogenesis with respect to NGP and stem cells are discussed above, it is understood that neogenesis may apply to any other neural cell types. Thus, the above paragraphs are equally applicable if the terms "NGP" and "stem cell" are substituted with any other neural cell type.

We describe herein our experiments using MC4R antagonists and the surprising finding that agents decreasing the activity of MC4R, exemplified by e.g. HS014 and HS028, induce proliferation of adult neural precursor cells *in vitro* as well as *in vivo.* We also show that agents decreasing the activity of MC4R give a neurorestorative effect in the 6-OHDA rat model of Parkinson's disease.

For any of the uses in treating a CNS disorder of the invention, administration of the agent(s) decreasing the activity of MC4R may be is an effective dosage range for the treatment of a desired disease. The determination of an effective amount of such an agent to be administered is within the skill of one of ordinary skill in the art and will be routine to those persons skilled in the art, based on the teachings disclosed here. The amount of MC4R activity decreasing agent to be administered will depend upon the exact size and condition of the patient, but could be between 0.1 to 10 ng/kg/day, between 1-100 ng/kg/day, between 5-100 ng/kg/day, between 20-500 ng/kg/day, between 100-1000 ng/kg/day, between 0.5 ug to 20 ug/kg/day, between 2-50 ug/kg/day, between 5-100 ug/kg/day, between 50-500 ug/kg/day, between 500-1000 ug/kg/day, between 1-5 mg/kg/day, between 5-20 mg/kg/day or higher. In another method of dosage, the MC4R may be administered so that a target tissue achieves a modulator concentration of 0.0001nM to 500 nM, 0.001nR4 to 500 nM, 0.01nM to 500 nM, 0.1nm to 500 nM, 0.1 nM to 100 nM, or at least 1 nM, at least 50 nM, at least 100 nM, or at least 500 nM. Preferred dosages include systemic administration of at least 10 mg twice a week or at least 25 mg twice a week; systemic administration of at least 0.04 mg/kg/week, at least 0.08 mg/kg/week, at least 0.24 mg/kg/week, at least 36 mg/kg/week, or at least 48 mg/kg/week; systemic administration of at least 22 mcg twice a week or 44 mcg twice a week; or systemic administration of at least 3-10 mg/kg once a month. Particularly preferred dosage ranges are 0.04 mg/kg to 4 mg/kg and 0.05 mg/kg to 5 mg/kg. These dosages may be increased 10x, 100x or 1000x in transdermal or topical applications.

Weight increase in a subject as a response to administration of an agent decreasing the activity of MC4R may be used as a surrogate marker for an effective dose for the stimulation of CNS-neogenesis and the treatment of a desired disease, such as a neurodegenerative disease such as Parkinson's. Even the lowest dose of the agent capable of eliciting a significant weight gain is an effective dose for stimulation of CNS-neogenesis and the treatment of CNS-disorders. From this dose, the optimal effective dose may easily be fine-tuned higher or lower by observing the subject's symptoms and occurrence of any side-effects. The weight gain may be monitored by weighing the subject at regular intervals during the administration. The significant weight gain may be determined by comparing the average weight gain in a treated group of subjects to the average weight gain a group administered a placebo compound. The weight gain may also be determined relative to the pre-administration weight in the same subject. The weight gain may be expressed in terms of percentage increase relative to control or initial body weight. Preferred thresholds for significant weight gain are at least 2%, at least 5% , at least 10%, or more than 10% such as 1%, 2%, 3%, 4%, 5 %, 6%, 7 %, 8%, 9%, 10 %, 12 %, 15 %, 20 %, 25 %, 30 %, 40 % and 50 %. The weight gain may also be detected by statistical methods, such as Student's t-test. Preferred significant weight gain thresholds are p≤0.05, or p≤0.2, such as p-values of 0.2, 0.1, 0.05, 0.01 and 0.001.

### EXAMPLES

### Example 1 Preparation of the LVW and Neurosphere Culture Procedure

The anterior lateral wall of the lateral ventricle wall (LVW) of 5 week old mice was enzymatically dissociated at 37°C for 20 min in 0.8 mg/mL hyaluronidase and 0.5 mg/mL trypsin in DMEM containing 4.5 mg/mL glucose and 80 units/mL DNase. The cells were diluted in 5 mL of DMEM/F12 medium (containing B27 supplement, 125 mM HEPES Ph 7.4, 100 units/mL penicillin, and 100 micrograms/mL streptomycin). After passing through a 70 micron strainer, the cells were pelleted at 240 x g for 5 minutes, washed and recentrifuged. The supernatant was subsequently removed and the cells resuspended in medium supplemented with 20 nanogram/mL EGF, plated out in culture dishes and incubated at 37°C. Neurosphere cultures were ready to be split approximately 7 days after plating.

To split the neurosphere cultures, neurospheres were collected by centrifugation at 240 x g for 5 minutes. The supernatant was discarded and the neurospheres were resuspended in 0.5 mL Trypsin/EDTA in HBSS (1 x) and incubated at 37°C for 2 minutes. During this period, the neurospheres were triturated gently to aid dissociation. Following a further 2 min incubation at 37°C and trituration, 2 volumes of DMEM-F12+B27 medium were added. The cells were pelleted at 220 x g for 4 min and resuspended in DMEM/F12+B27-medium containing 10 ng/mL EGF and 5 ng/mL bFGF. Subsequently the cultures were plated out and incubated at 37°C.

### Example 2 Expression of MC4R; RT-PCR analysis

Neurospheres were prepared from the LVW as stated in example 1 above. Three days after the first split, the neurospheres were harvested and total RNA was isolated using QIAGEN's RNeasy Mini Kit according to the manufacturer's instructions. LVW and rest of brain total RNA was prepared in identical fashion to that of neurosphere total RNA. Prior to the RT-PCR, total RNA was DNase (Ambion) treated (1 unit for each 5 micrograms of total RNA) at 37°C for 15 min, followed by heat inactivation at 75°C for 10 min. Invitrogen's One-Step RT-PCR Kit was used to detect the presence of mRNA corresponding to the MC4R. Briefly, 12.5 ng of total RNA was used in each reaction, with an annealing temperature of 58°C. To further ensure that genomic contamination of the total RNA did not give rise to false positive results, an identical reaction with Taq polymerase alone was run in parallel with the experimental RT-PCR. The reactions were electrophoresed on a 1.0% agarose gel containing ethidium bromide and bands were visualized under UV light. Bands corresponding to the estimated length of PCR products of the desired genes were cloned into the cloning vector pGEM-Teasy. Constructs were sequenced to verify their identity. Primer sequences for the *Mus musculus* MC4R are shown below.

| **Receptor** | **Primer** | **Band size (bp)** |
|---|---|---|
| MC4R | Fw: 5'-ggatacggatgcccagagct-3' (SEQ ID NO:1) | 327 |
| MC4R | Rev: 5'-gccatcaggaacatgtggaca-3' (SEQ ID NO:2) | |

These studies investigated the mRNA expression pattern of MC4R in the adult mouse brain. The results indicated that MC4R is expressed in neurogenic regions of adult mouse brain. Using RT-PCR, it was found that the MC4R mRNA is expressed in both the lateral ventricle wall tissue (including the ependymal layer) and in neurospheres derived from cultured neural stems cells (NSCs) derived from this tissue (Table 2).

**Table 2: Expression of MC4R mRNA in adult mouse brain estimated by RT-PCR (on a 1-3 scale) in neurogenic tissue.**

| ***RT-PCR*** | **Murine MC4R** |
|---|---|
| Neurospheres | ++ |
| Lateral ventricle wall | +++ |
| Rest of brain | ++ |

### Example 3 MC4R antagonist treatment of cells from example 1 grown in suspension medium or as adherent culture

HS014 and HS028 were purchased from Sigma Aldrich.

Cells were harvested split as in example 1, except for omission of the growth factors in the media, and seeded as suspension cells, at a density of 10,000 cells/well on 96-well plates in DMEM/F12 supplemented with 1, 10 or 100 nM MC4R antagonist or without (control cells). Alternatively, adherent cells were seeded at a density of 30,000 cells /well on poly-D-lysine-coated 96-well plates in DMEM/F12 supplemented with 1% fetal calf serum (FCS). When the cells had adhered (after 4 hours), the medium was changed to serum-free medium, and 1, 10 or 100 nM HS014, HS028, or other test compounds were added.

### In vitro proliferation assays

Intracellular ATP levels have previously been shown to correlate to cell number [52]. The following experiment was performed in quadruplicate. HS014 or HS028 were added and cells were incubated at 37°C for 3 days. Cells were subsequently lysed. Intracellular ATP was measured using a Cell Viability kit SL according to the manufacturer's instructions (#188-441, BioThema, Sweden). Results were repeatable and statistically significant. The ATP-assay was performed in suspension culture setting (see above)

DNA synthesis is commonly used to measure cell proliferation. For such measurements, ³H-thymidine is traditionally used to label the DNA of mitotically active cells. In this experiment, ³H-thymidine was replaced by 5-bromo-2-deoxyuridine (BrdU), and was added together with the compounds at 10 µM concentration. The BrdU assay was performed in adherent cell culture setting (see above), and the cells were incubated with the compounds for 3 days. After incorporation if the thymidine analogue into DNA, BrdU was detected by immunoassay. The ELISA kit was commercially available from Roche, Germany.

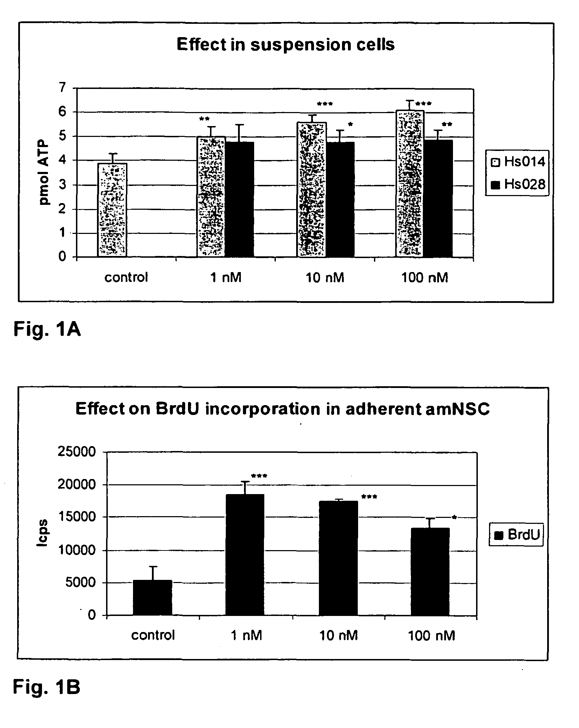

It was found that the MC4R antagonists HS014 and HS028 induce *in vitro* proliferation of adult neural stem cells. Using the ATP assay, increases in intracellular ATP levels (and hence cell numbers) were seen in MC4R antagonist-treated suspension cultures (1-100 nM concentration) (Fig 1a). To confirm proliferation, incorporation studies using the proliferative marker BrdU were performed. Increases in BrdU incorporation were seen in MC4R antagonist-treated adherent cell cultures (1-100nM concentration) Fig 1B. The increases in ATP and BrdU incorporation were determined to be statistically significant (FIG. 1a and b) by student's two-tailed unpaired t-test. In the same ATP-assay, treatment with the endogenous MC4R antagonist peptide analogues Agouti (1-40) amide and Agouti (87-132) likewise caused statistically significant increases in proliferation (1.3-1.4 times control at 100 nM, and 1.6 times control at 400 nM, respectively).

In conclusion, compounds decreasing the activity of MC4R are able to increase the cell number and induce proliferation of neuronal stem/progenitor cells in vitro.

### Example 4 In vivo proliferation experiment

To further characterize MC4R antagonist-stimulatiow of neogenesis, *in vivo* studies were performed. The effect of HS014 on proliferation (BrdU incorporation) was tested over a two week period at doses ranging from 6.25 to 625 ng/24h. Administration was done by i.c.v. infusion into the right lateral ventricle. BrdU was administered together with HS014 i.c.v. The animal's body weight was measured throughout the experiment. After termination of the experiment, BrdU expression was detected by immunohistochemistry on brain sections from the subependymal layer of the lateral ventricle wall as well as on the sub granular cells in dentate gyrus.

For these studies, HS014 was dissolved to 3,33 microM in artificial CSF (148mM NaCl, 3mM KCl, 1.4mM CaCl₂, 0.8mM MgCl₂, 1.5mM Na₂HPO₄, 0.2mM NaH₂PO₄, pH 7,4 sterile filtered before use and also containing 1mg/mL BrdU, 50µg/mL Gentamycin, and 100 microgram/mL rat serum albumin. Solutions of BrdU 1mg/mL, 100 microgram/mL rat serum albumin, 50 microgram/mL gentamycin, HS014 in artificial CSF and were prepared. Pumps were filled via tubing with 0.9% NaCl, connected to flow moderators and stored at 37°C overnight.

A dose range of 1-100 nM of both HS014 and HS028 was found to be effective in increasing the number of adult neural stem cells cell compared to controls in *in vitro* assays (see Example 3). To estimate a dose that would be effective in increasing neural stem cell number in vivo by administering HS014 by continuous infusion into the lateral ventricle of adult rats, the volume of cerebrospinal fluid (CSF) and the rate of turnover were used to give a CSF concentration in the range of 1-100 nM, resulting in pump concentrations delivering of 6.25, 62.5, and 625 ng/day. As an additional marker for understanding whether a dose that is effective in eliciting a response controlled by the central nervous system, weight gain was also monitored throughout the experiment by weighing the animals. MC4R antagonists induce weight gain [53] acting on areas of the brain known to influence food intake and metabolism, particularly the arcuate, paraventricular, lateral, and ventromedial nuclei of the hypothalamus [53, 54].

Adult rats weighing approximately 290 grams were deeply anaesthetized with isoflurane and a cannula was stereotaxically implanted for infusions into the right lateral ventricle (coordinates: AP -0.8 mm; L -1.7 mm and DV -4.0 mm, relative bregma) (ALZET brain infusion kit I). The cannula was secured to the skull with dental cement in anchoring screws. The infusion kit was connected to a subcutaneous implanted mini-osmotic pump (ALZET 2002) placed in the mid-scapular region of the animal. The rats (5, 6, and 6 animals in the respective treated groups, 6 in the control) received 0.5 microL/hour of the solution (6.25, 62.5, or 625 ng/day HS014 for the treated groups) for two weeks and were subsequently sacrificed.

At the end of the experiment the rats were anaesthetized with sodium pentobarbital (120mg, i.p.) and transcardially perfused with 90 ml of 0.9 % NaCl and 4% PFA solution. The brain was removed, frozen in isopentane at -40°C and stored at -20°C until cryosectioned. The brains were sectioned 12µm coronally on a cryostat, both lateral ventricle, bregma 1.6mm (three adjacent section on each glasses) and dentate gyrus, region spanning from bregma -2.56 to -4.16 mm according to Paxinos rat brain atlas (two adjacent section on each glasses). The sections were thaw mounted on superfrost microscope slides and stored at -20°C until required. For BrdU immunhistochemistry the slides were denatured in 2M HCl and unspecific binding blocked by incubating with 10% goat serum over night. This was followed by application of rat monoclonal anti-BrdU (Harlan) 1/100 with 0.1% tween-20 and PBS incubated 90 min a humidified chamber. After a washing in PBS the secondary antibody anti rat IgG biotinylated (Vector) 1/200 was applied and incubated for 1 hr at room temperature. As detection, Vectastain Elite (Vector) 1/100 was used and finally, developing was done by Sigma fast DAB tablet monitored in microscope, sections were then counterstained by HTX followed by dehydration and clearing.

Quantification was performed using a Nikon eclipse E6000 microscope at a magnification of 40x. An observer blind with regard to the treatment condition conducted analyses. Three adjacent sections from the ipsilateral side of the SVZ at bregma 1.6mm were evaluated. All BrdU positive cells within the sub ependymal layer were counted. All counts were pooled together for each rat and are reported as mean number of cells per square.

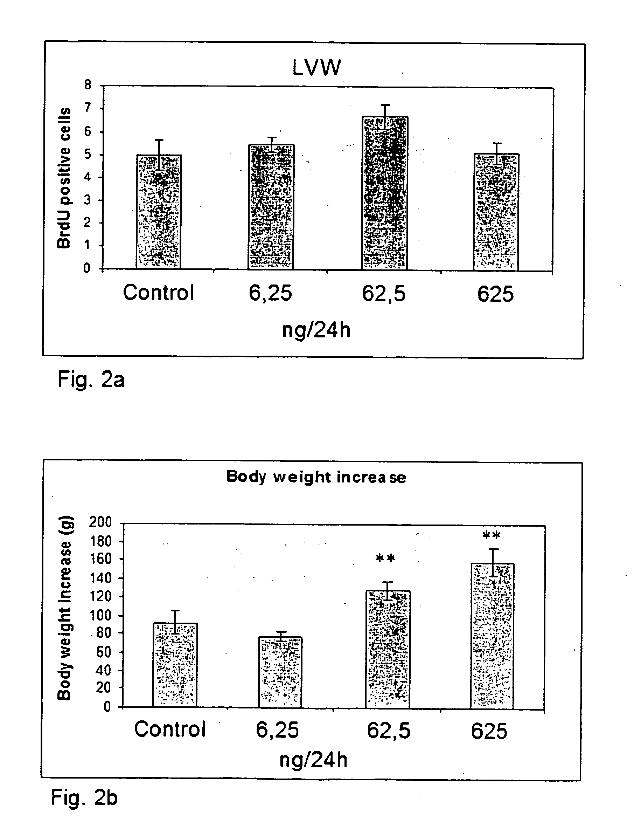

The results for BrdU counts are shown in Fig. 2A. The effect of HS014 on neuronal stem/progenitor cell proliferation was greatest at a dose of 62.5 ng/day. The weight gain (Fig. 2B), however, was increased in a dose-dependent manner and was significant first at a dose of 62.5 ng/day, being greatest at 625 ng/day. The weight gain indicates a physiological effect also in the hypothalamus.

In conclusion, an agent decreasing the activity of MC4R is able to increase the proliferation of neuronal stem/progenitor cells in vivo. Significantly, we find unexpectedly that weight gain can be used as a surrogate marker for a pharmacological CNS-effect when treating a subject with an agent resulting in a decrease in MC4R activity. Even a dose eliciting a modest weight gain is sufficient in inducing the proliferation of neuronal stem/progenitor cells.

### Example 5 In vivo Parkinson's disease model experiment

In this study, the neurorestorative action of HS014 (from Sigma Aldrich) was investigated in the 6-hydroxydopamine (6-OHDA)-lesioned rat model of Parkinson's disease where the animals have a partial, unilateral loss of dopaminergic neurons. The lesion is induced by a single injection of 6-OHDA in the median forebrain bundle (see below). Upon amphetamine stimulation, the lesioned animals will display ipsiversive rotational behavior due to the imbalance in dopamine release resulting from the unilateral loss of dopaminergic cells. This allows the functional effects of a compound tested in this model to be quantified by counting the number of amphetamine-induced rotations [55]. Animals with complete lesion will exhibit contraversive rotational behavior after apomorphine stimulation, making it possible to exclude any fully lesioned animals from the study.

### Preparation of 6-OHDA-lesioned rat model of Parkinson's disease

Male Sprague-Dawley rats weighing 280-320 g were housed in a temperature-controlled room under a 12 h light / dark cycle with free access to food and water. Thirty minutes prior to surgery, animals were injected intraperitoneally with pargyline (5 mg/kg) and desipramine (25 mg/kg). Rats were then placed in a stereotactic frame under general anaesthesia (Halothane). A small bur-hole was made in the right side of the skull. Each animal was given a unilateral injection of 4 µg, 6-OHDA (in 2 µl sterile water with 0.1% ascorbic acid) into the right medial forebrain bundle at co-ordinates -2.8mm from bregma, 2 mm lateral to the midline, and 8.6 mm below skull according to the atlas of Paxinos and Watson [56]. The 6-OHDA injection was made over a 5 min period using a 5 µl Hamilton syringe. The rats were allowed to recover for 5 weeks following the lesion. The animals were then implanted with an Alzet minipump attached to a cannula to allow infusion of HS014 or vehicle into the right lateral ventricle over a period of 2 weeks. The Alzet minipump was removed 1 week later.

### Preparation of pump

Materials: Brain infusion kit II (ALZET, Cupertiono, CA); ALZET® osmotic minipump model 2002 (volume of pump: 200 µl, pumping rate: 0.5 µl/h (=12 µl/d) for 14 days) The pump was filled with 200 µl of solution containing either vehicle (148mM NaCl, 3mM KCl, 1.4 mM CaCl₂, 0.8mM MgCl₂, 1.5mM Na₂HPO₄, 0.2mM NaH₂PO₄, pH 7.4, 50µg/ml Gentamycin and 100µg/ml rat serum albumin), or compound solution (vehicle plus 5.21 µg/ml HS014, resulting in a dose of 62.5 ng/day), connected via tubing to a flow moderator and incubated in NaCl (0.9%) solution in a water bath (37°C) for 2-5 hrs prior to implantation.

### Study design

24 rats were divided into 2 groups, individual rats being randomly assigned to a treatment group based on their baseline rotation (5 weeks post lesion) so they were equally distributed. Thus, there were 12 partially lesioned rats per group. The groups received vehicle or drug treatment via an implanted Alzet minipump for 2 weeks. The rats were then left without treatment for a further 2 weeks after which they were sacrificed. During the study the animals received 0.05 mg/kg s.c. apomorphine and 5 mg/kg i.p. amphetamine before the pump implant and then once every week, to measure rotational response. Rotational activity was measured by counting the total number of turns for the duration of the experiment. (i.e. 60 min following apomorphine or amphetamine administration). Body weight was also recorded weekly.

### Dosing rationale

A dose that stimulated both an increase in neural stem cell number in the subventricular zone of the lateral ventricle and elicited an increase in weight gain greater than that of control animals (Example 4) was selected as a dose to administer.

### Effect of MC4R activity decreasing agent on amphetamine-induced rotation

Any fully lesioned animals, determined by rotatory behavior following apomorphine stimulation, were excluded. Prior to pump implantation, amphetamine induced 365.75 ± 48 and 300.417 ± 44 (Average ±S.E.M.) net ipsiversive rotations in the vehicle-treated and HS014 groups, respectively. The difference between the baseline levels of the groups was not significant (P > 0.05; unpaired t-test). In the vehicle group, weekly amphetamine challenges did not show any reduction in net ipsiversive rotations over the course of the study compared to the pre-pump response (all P < 0.05; one-way repeated measures ANOVA followed by Dunnett's test, Fig. 4).

In the HS014 group, weekly amphetamine challenges did however demonstrate a significant reduction in net ipsiversive rotations at weeks 1 - 4 inclusive compared to the pre-pump response (all P < 0.01; one-way repeated measures ANOVA followed by Dunnett's test, Fig. 4). The magnitude of the reduction at week 4 was such that the rotation reduced to approximately 30% compared to pre-pump levels. Comparison of the vehicle group with the HS014 group demonstrated a significant difference in ipsiversive rotations at week 2 (P < 0.05; unpaired t-test; Fig. 4).

The results showed that administration of a MC4R activity decreasing agent (the MC4R antagonist HS014) induced a significant improvement in a model of Parkinson's disease. Remarkably, the improvement was maintained for at least two weeks after administration of the compound was stopped. A long lasting normalization of the rotational behavior can be attributed to a neurorestorative effect. In other words, the effect seems to be a plastic rather than a transitory effect and suggested that the MC4-antagonist was indeed triggering a stable change in the brain by improving the course of the pathology rather than just exerting a symptomatic and acute effect. The fact that the same compound was able to induce proliferation of neural stem cells in vitro and in vivo, together with the sustained effect observed in this Parkinson's model, indicated that treatment of Parkinson's with a MC4R activity decreasing agent acts via the mechanism of CNS neogenesis.

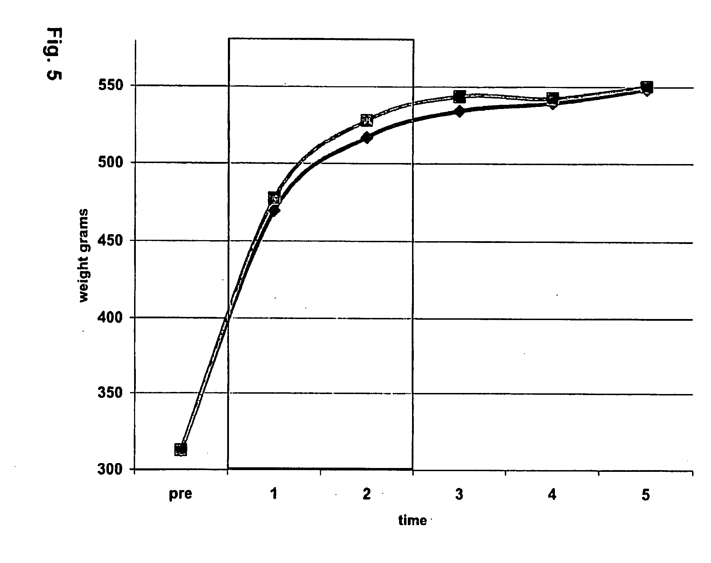

The compound administration caused a small but noticeable increase in body weight of the animals, which was transient in nature (Figure 5). The results show that a slight increase in body weight can be used as a surrogate marker for a dose of a MC4R activity decreasing agent effective for treatment of a degenerative CNS disorder.

### Example 6 In vivo Parkinson's disease model experiment #2

In this study, the neurorestorative action of Tran12e and Xi14g are investigated in the 6-hydroxydopamine (6-OHDA)-lesioned rat model of Parkinson's disease where the animals have a partial, unilateral loss of dopaminergic neurons. The lesion is induced by a single injection of 6-OHDA in the median forebrain bundle (see below). Upon amphetamine stimulation, the lesioned animals will display ipsiversive rotational behavior due to the imbalance in dopamine release resulting from the unilateral loss of dopaminergic cells. This allows the functional effects of a compound tested in this model to be quantified by counting the number of amphetamine-induced rotations [55]. Animals with complete lesion will exhibit contraversive rotational behavior after apomorphine stimulation, making it possible to exclude any fully lesioned animals from the study.

### Preparation of 6-OHDA-lesioned rat model of Parkinson's disease

Male Sprague-Dawley rats weighing 280-320 g are housed in a temperature-controlled room under a 12 h light / dark cycle with free access to food and water. Thirty minutes prior to surgery, animals are injected intraperitoneally with pargyline (5 mg/kg) and desipramine (25 mg/kg). Rats are then placed in a stereotactic frame under general anaesthesia (Halothane). A small bur-hole is made in the right side of the skull. Each animal is given a unilateral injection of 4 µg 6-OHDA (in 2 µl sterile water with 0.1% ascorbic acid) into the right medial forebrain bundle at co-ordinates -2.8mm from bregma, 2 mm lateral to the midline, and 8.6 mm below skull according to the atlas of Paxinos and Watson [56]. The 6-OHDA injection is made over a 5 min period using a 5 µl Hamilton syringe. The rats are allowed to recover for 5 weeks following the lesion.

### Preparation of administration solution

Test compounds are dissolved in vehicle consisting of sterile phosphate-buffered saline just prior to administration, and sterile filtetered with 0.22 µm filter.

### Study design

36 rats are divided into 3 groups, individual rats being randomly assigned to a treatment groups based on their baseline rotation (5 weeks post lesion) so they are equally distributed. Thus, there are 12 partially lesioned rats per group. The groups receive vehicle or drug treatment via systemic administration for two weeks. The rats are then left without treatment for a further 2 weeks after which they are sacrificed. During the study the animals receive 0.05mg/kg s.c. apomorphine and 5 mg/kg i.p. amphetamine before the pump implant and then once every week, to measure rotational response. Rotational activity is measured by counting the total number of turns for the duration of the experiment. (i.e. 60 min following apomorphine or amphetamine administration). Body weight is also recorded weekly.

### Dosing rationale

A pre-study akin to example 4 is performed to find the lowest dose for each compound that induces a significant weight increase in the treated group. Ten groups with 6 rats each are systemically administered the compounds from 10 mg/kg/day to 10 pg/kg/day with 10-fold differences between groups, for two weeks. The lowest dose that induces a significant weight increase is selected for the 6-OHDA model.

### Effect of MC4R activity decreasing agents on amphetamine-induced rotation

Any fully lesioned animals, determined by rotatory behavior following apomorphine stimulation, are excluded. Prior to treatment, amphetamine induces approximately similar net ipsiversive rotations in the vehicle and treatment groups, respectively. The difference between the baseline levels of the groups is not significant (P > 0.05; unpaired t-test). In the vehicle group, weekly amphetamine challenges does not show any reduction in net ipsiversive rotations over the course of the study compared to the pre-treatment response (all P < 0.05; one-way repeated measures ANOVA followed by Dunnett's test).

In the treatment groups, weekly amphetamine challenges do however demonstrate a significant reduction in net ipsiversive rotations at weeks 1 - 4 inclusive compared to the pre-treatment response (P < 0.05; one-way repeated measures ANOVA followed by Dunnett's test). The magnitude of the reduction at week 4 is such that the rotation reduced to approximately 30%-50% compared to pre-treatment levels. Comparison of the vehicle group with the treatment groups demonstrates a significant difference in ipsiversive rotations at week 2 (P < 0.05; unpaired t-test).

The results show that administration of MC4R activity decreasing agents induce a significant improvement in a model of Parkinson's disease. Remarkably, the improvement is maintained for at least two weeks after administration of the compound is stopped. A long lasting normalization of the rotational behavior can be attributed to a neurorestorative effect. In other words, the effect seems to be a plastic rather than a transitory effect and suggests that the MC4-antagonist is indeed triggering a stable change in the brain by improving the course of the pathology rather than just exerting a symptomatic and acute effect, indicating effect via CNS-neogenesis.

The compound administration also causes a small but noticeable increase in body weight of the animals, which is transient in nature. The results shows that a slight increase in body weight can be used as a surrogate marker for a dose of a MC4R activity decreasing agent effective for treatment of a degenerative CNS disorder.

### Example 7 Further In vivo Parkinson's disease model experiment

The experiment is performed essentially as repetition of Examples 5-6, but using different compounds that substantially decrease the activity of MC4R-receptor in its natural setting. The compounds are antagonists, inverse agonists, inhibitors and/or other agents as specified in the description. Compounds used are exemplified in Table 1. For the compounds that are able to penetrate the blood-brain barrier, the administration is done systemically as in example 6. For compounds that do not pass the blood-brain barrier, intracereboventricular administration as in example 5 is used.

Similar results as in examples 5 and 6 are obtained, which shows that the effect in Parkinson's disease is general for compounds with substantial MC4R-activity decreasing properties.

### REFERENCES

1. Altman, J. and G.D. Das, Autoradiographic and histological evidence of postnatal hippocampal neurogenesis in rats. J Comp Neurol, 1965. 124(3): p. 319-35.
2. Altman, J. and G.D. Das, Postnatal neurogenesis in the guinea-pig. Nature, 1967. 214(93): p. 1098-101.
3. Momma, S., C.B. Johansson, and J. Frisen, Get to know your stem cells. Curr Opin Neurobiol, 2000. 10(1): p. 45-9.
4. Kuhn, H.G. and C.N. Svendsen, Origins, functions, and potential of adult neural stem cells. Bioessays, 1999. 21(8): p. 625-30.
5. Doetsch, F., et al., Subventricular zone astrocytes are neural stem cells in the adult mammalian brain. Cell, 1999. 97(6): p. 703-16.
6. Johansson, C.B., et al., Identification of a neural stem cell in the adult mammalian central nervous system. Cell, 1999. 96(1): p. 25-34.
7. Kempermann, G., H.G. Kuhn, and F.H. Gage, Experience-induced neurogenesis in the senescent dentate gyrus. J Neurosci, 1998. 18(9): p. 3206-12.
8. Palmer, T.D., et al., Fibroblast growth factor-2 activates a latent neurogenic program in neural stem cells from diverse regions of the adult CNS. J Neurosci, 1999. 19(19): p. 8487-97.
9. Lois, C. and A. Alvarez-Buylla, Proliferating subventricular zone cells in the adult mammalian forebrain can differentiate into neurons and glia. Proc Natl Acad Sci U S A, 1993. 90(5): p. 2074-7.
10. Biebl, M., et al., Analysis of neurogenesis and programmed cell death reveals a self-renewing capacity in the adult rat brain. Neurosci Lett, 2000. 291(1): p. 17-20.
11. Snyder, E.Y., et al., Multipotent neural precursors can differentiate toward replacement of neurons undergoing targeted apoptotic degeneration in adult mouse neocortex. Proc Natl Acad Sci U S A, 1997. 94(21): p. 11663-8.
12. Magavi, S.S., B.R. Leavitt, and J.D. Macklis, Induction of neurogenesis in the neocortex of adult mice. Nature, 2000. 405(6789): p. 951-5.
13. Johansson, C.B., et al., Neural stem cells in the adult human brain. Exp Cell Res, 1999. 253(2): p. 733-6.
14. McKay, R., Stem cells in the central nervous system. Science, 1997. 276(5309): p. 66-71.
15. Rajan, P. and R.D. McKay, Multiple routes to astrocytic differentiation in the CNS. J Neurosci, 1998. 18(10): p. 3620-9.
16. Johe, K.K., et al., Single factors direct the differentiation of stem cells from the fetal and adult central nervous system. Genes Dev, 1996. 10(24): p. 3129-40.
17. Williams, B.P., et al., A PDGF-regulated immediate early gene response initiates neuronal differentiation in ventricular zone progenitor cells. Neuron, 1997. 18(4): p. 553-62.
18. Pluchino, S., et al., Neurosphere-derived multipotent precursors promote neuroprotection by an immunomodulatory mechanism. Nature. 2005 Jul 14;436(7048):266-71
19. Herman, J.P. and N.D. Abrous, Dopaminergic neural grafts after fifteen years: results and perspectives. Prog Neurobiol, 1994. 44(1): p. 1-35.
20. Bjorklund, A. and O. Lindvall, Cell replacement therapies for central nervous system disorders. Nat Neurosci, 2000. 3(6): p. 537-44.
21. Kuhn, H.G., et al., Epidermal growth factor and fibroblast growth factor-2 have different effects on neural progenitors in the adult rat brain. J Neurosci, 1997. 17(15): p. 5820-9.
22. Craig, C.G., et al., In vivo growth factor expansion of endogenous subependymal neural precursor cell populations in the adult mouse brain. J Neurosci, 1996. 16(8): p. 2649-58.
23. Pencea, V., et al., Infusion of brain-derived neurotrophic factor into the lateral ventricle of the adult rat leads to new neurons in the parenchyma of the striatum, septum, thalamus, and hypothalamus. J Neurosci, 2001. 21(17): p. 6706-17.
24. Fallon, J., et al., In vivo induction of massive proliferation, directed migration, and differentiation of neural cells in the adult mammalian brain. Proc Natl Acad Sci U S A, 2000. 97(26): p. 14686-91.
25. Wikberg, J.E., Melanocortin receptors: perspectives for novel drugs. Eur J Pharmacol, 1999. 375(1-3): p. 295-310.
26. Wikberg, J.E., et al., New aspects on the melanocortins and their receptors. Pharmacol Res, 2000. 42(5): p. 393-420.
27. Chaki, S. and S. Okuyama, Involvement of melanocortin-4 receptor in anxiety and depression. Peptides, 2005. 26(10): p. 1952-64.
28. Gantz, I., et al., Molecular cloning, expression, and gene localization of a fourth melanocortin receptor. J Biol Chem, 1993. 268(20): p. 15174-9.
29. Kishi, T., et al., Expression of melanocortin 4 receptor mRNA in the central nervous system of the rat. J Comp Neurol, 2003. 457(3): p. 213-35.
30. Mountjoy, K.G., et al., Localization of the melanocortin-4 receptor (MC4-R) in neuroendocrine and autonomic control circuits in the brain. Mol Endocrinol, 1994. 8(10): p. 1298-308.
31. Chai, B.X., et al., Inverse agonist activity of agouti and agouti-related protein. Peptides, 2003. 24(4): p. 603-9.
32. Manganas, L. N., et al., Magnetic resocance spectroscopy identifies neural progenitor cells in the live human brain. Science. 318(5852):980-5
33. Kask, A., et al., Selective antagonist for the melanocortin 4 receptor (HS014) increases food intake in free-feeding rats. Biochem Biophys Res Commun, 1998. 245(1): p. 90-3.
34. Kask, A., et al., Long-term administration of MC4 receptor antagonist HS014 causes hyperphagia and obesity in rats. Neuroreport, 1999. 10(4): p. 707-11.
35. Pontillo, J., et al., A potent and selective nonpeptide antagonist of the melanocortin-4 receptor induces food intake in satiated mice. Bioorg Med Chem Lett, 2005. 15(10): p. 2541-6.
36. Chen, C., et al., 4-{(2R)-[3-Aminopropionylamido]-3-(2,4-dichlorophenyl)propionyl}-1- {2-[(2- thienyl)ethylaminomethyl]phenyl}piperazine as a potent and selective melanocortin-4 receptor antagonist--design, synthesis, and characterization. J Med Chem, 2004. 47(27): p. 6821-30.
37. Xi, N., et al., Synthesis of novel melanocortin 4 receptor agonists and antagonists 37. containing a succinamide core. Bioorg Med Chem Lett, 2004. 14(2): p. 377-81.
38. Schioth, H.B., et al., Further pharmacological characterization of the selective melanocortin 4 receptor antagonist HS014: comparison with SHU9119. Neuropeptides, 1999. 33(3): p. 191-6.
39. Skuladottir, G.V., et al., Long term orexigenic effect of a novel melanocortin 4 receptor selective antagonist. Br J Pharmacol, 1999. 126(1): p. 27-34.
40. Kask, A., et al., Discovery of a novel superpotent and selective melanocortin-4 receptor antagonist (HS024): evaluation in vitro and in vivo. Endocrinology, 1998. 139(12): p. 5006-14.
41. Chen, C., et al., Discovery of J-[2-[(1S)-(3-dimethylaminopropionyl)amino-2-methylpropyl]-4-methylphenyl]-4-[(2R)-methyl-3-(4-chlorophenyl)-propionyl]piperazine as an orally active antagonist of the melanocortin-4 receptor for the potential treatment of cachexia. J Med Chem, 2007. 50(22): p. 5249-52.
42. Chen, C.W., et al., Synthesis and characterization of trans-4-(4-chlorophenyl)pyrrolidine-3-carboxamides of piperazinecyclohexanes as ligands for the melanocortin-4 receptor. Bioorg Med Chem Lett, 2007. 17(24): p. 6825-31.
43. Chen, C., et al., Identification and characterization of pyrrolidine diastereoisomers as potent functional agonists and antagonists of the human melanocortin-4 receptor. Bioorg Med Chem Lett, 2007.
44. Tran, J.A., et al., Pyrrolidinones as orally bioavailable antagonists of the human melanocortin-4 receptor with anti-cachectic activity. Bioorg Med Chem, 2007. 15(15): p. 5166-76.
45. Vos, T.J., et al., Identification of 2-[2-[2-(5-bromo-2-methoxyphenyl)-ethyl]-3-fluorophenyl]-4,5-dihydro-1H-imidazole (ML00253764), a small molecule melanocortin 4 receptor antagonist that effectively reduces tumor-induced weight loss in a mouse model. J Med Chem, 2004. 47(7): p. 1602-4.
46. Chaki, S., et al., MCL0042: a nonpeptidic MC4 receptor antagonist and serotonin reuptake inhibitor with anxiolytic- and antidepressant-like activity. Pharmacol Biochem Behav, 2005. 82(4): p. 621-6.
47. Nozawa, D., et al., Identification of arginine analogues as antagonists and agonists for the melanocortin-4 receptor. Chem Pharm Bull (Tokyo), 2007. 55(8): p. 1232-9.
48. Nozawa, D., et al., Novel piperazines: potent melanocortin-4 receptor antagonists with anxiolytic-like activity. Bioorg Med Chem, 2007. 15(6): p. 2375-85.
49. Ying, J., et al., Design, synthesis, and biological evaluation of new cyclic melanotropin peptide analogues selective for the human melanocortin-4 receptor. J Med Chem, 2006. 49(23): p. 6888-96.
50. Van der Ploeg, L.H., et al., Design and synthesis of (ant)-agonists that alter appetite and adiposity. Prog Brain Res, 2006. 153: p. 107-18.
51. Schiöth et al., Discovery of novel melanocortin4 receptor selective MSH analogues. Br J Pharmacol. 1998 May; 124(1):75-82
52. Crouch, S.P., et al., The use of ATP bioluminescence as a measure of cell proliferation and cytotoxicity. J Immunol Methods, 1993. 160(1): p. 81-8.
53. Foster, A.C. and C. Chen, Melanocortin-4 receptor antagonists as potential therapeutics in the treatment of cachexia. Curr Top Med Chem, 2007. 7(11): p. 1131-6.
54. Joppa, M.A., et al., Central infusion of the melanocortin receptor antagonist agouti-related peptide (AgRP(83-132)) prevents cachexia-related symptoms induced by radiation and colon-26 tumors in mice. Peptides, 2007. 28(3): p. 636-42.
55. Ungerstedt, U. and G.W. Arbuthnott, Quantitative recording of rotational behavior in rats after 6-hydroxy-dopamine lesions of the nigrostriatal dopamine system. Brain Res, 1970. 24(3): p. 485-93.
56. Paxinos G. and C. Watson. The Rat Brain in Stereotaxic Coordinates. Second Edition. New York: Academic Press, 1986.

## Claims

1. A MC4R activity decreasing agent for use in treating a central nervous system (CNS) disorder in a subject wherein said MC4R activity decreasing agent is to be administered to the subject in an amount sufficient to alleviate a symptom of said disorder, wherein the CNS disorder is
Parkinson's disease or Parkinsonian disorders;
and wherein said agent is
selected from the group consisting of HS014, HS028, compound 10,
compound Pontillo14c, compound Xi14a, compound Xi14b, compound Xi14c, compound Xi14d, compound Xi14e, compound Xi14f,
compound Xi14g, compound Xi14h, compound Xi 14i, compound Xi14j, HS024, Compound 10d, Compound 18v, Compound 13b-2,
Compound Tran12e, and combinations thereof.

2. The MC4R activity decreasing agent for use of claim 1 wherein the subject is also administered
a) one or more growth factors, or
b) one or more agents selected from the group consisting of antidepressants, anti-anxiety agents, anti-psychotic agents, anti-epilepsy agents, anti-Alzheimer's agents, and-Parkinson's agents, MAO inhibitors, serotonin-uptake blockers, noradrenaline uptake blockers, dopamine uptake blockers, dopamine agonists, L-DOPA, tranquilizers, sedatives, and lithium.

3. A MC4R activity decreasing agent for use according to claim 1 wherein said treating further comprises
monitoring an indicia of neogenesis in said subject by a non-invasive method to determine if the doses are sufficient; and
increasing said doses if said indicia of neogenesis is insufficient or decreasing said doses if said indicia of neogenesis is excessive.

4. The MC4R activity decreasing agent for use of claim 3 wherein said monitoring step comprises a step of monitoring a weight of said subject and wherein the sufficient dose is determined as the minimum dose that causes an increase in body weight of said subject above a set threshold compared to a second subject with a similar symptom the CNS disorder but not administered said composition.

5. The MC4R activity decreasing agent for use of claim 3 wherein said subject has a decreased number of dopaminergic neurons compared to a healthy subject before administration of said agent.

6. The MC4R activity decreasing agent for use of claim 1 wherein said Parkinson's disease or Parkinsonian disorders is a degenerative CNS disorder and said agent is administered for a period of time until said subject displays a desired reduction in symptoms, and wherein said subject shows a continued reduction in symptoms for a period of at least two weeks after the administration of said agent is stopped.

7. The MC4R activity decreasing agent for use of claim 6 wherein said treatment increases the proliferation of adult neural stem cells in the lateral ventricular wall.

8. The MC4R activity decreasing agent for use of claim 6 wherein the agent is administered to achieve a tissue concentration of 0.1 nM to 500 nM.

9. The MC4R activity decreasing agent for use of claim 6 wherein said agent is administered to a subject with decreased dopaminergic neurons in the brain.

## Patentansprüche

1. Die MC4R-Aktivität verringerndes Mittel zur Verwendung bei der Behandlung einer Erkrankung des zentralen Nervensystems (ZNS) bei einem Individuum, wobei das die MC4R-Aktivität verringernde Mittel dem Individuum in einer Menge zu verabreichen ist, die ausreicht, um ein Symptom der Erkrankung zu lindern, wobei die ZNS-Erkrankung
Parkinson-Krankheit oder Parkinson'sche Erkrankungen sind;
und wobei das Mittel
ausgewählt ist aus der Gruppe, die aus HS014, HS028, Verbindung 10, Verbindung Pontillo 14c, Verbindung Xi14a, Verbindung Xi14b, Verbindung Xi14c, Verbindung Xi14d, Verbindung Xi14e, Verbindung Xi14f, Verbindung Xi14g, Verbindung Xi14h, Verbindung Xi14i, Verbindung Xi14j, HS024, Verbindung 10d, Verbindung 18v, Verbindung 13b-2, Verbindung Tran 12e und Kombinationen davon besteht.

2. Die MC4R-Aktivität verringerndes Mittel zur Verwendung nach Anspruch 1, wobei dem Individuum auch verabreicht wird
a) ein oder mehrere Wachstumsfaktoren, oder
b) ein oder mehrere Mittel, die ausgewählt sind aus der Gruppe, die aus Antidepressiva, angstlösenden Mitteln, antipsychotischen Mitteln, antiepileptischen Mitteln, Mitteln gegen Alzheimer, Mitteln gegen Parkinson, MAO-Hemmern, Serotonin-Aufnahme-Blockern, Noradrenalin-Aufnahme-Blockern, Dopanin-Aufnahme-Blockern, Dopamin-Agonisten, L-DOPA, Tranquilizern, Sedativa und Lithium besteht.

3. Die MC4R-Aktivität verringerndes Mittel zur Verwendung nach Anspruch 1, wobei die Behandlung außerdem aufweist Überwachen eines Anzeichens für Neogenese bei dem Individuum durch ein nicht-invasives Verfahren, um zu bestimmen, ob die Dosen ausreichend sind; und
Erhöhen der Dosen, wenn das Anzeichen für Neogenese unzureichend ist, oder Verringern der Dosen, wenn das Anzeichen für Neogenese übermäßig ist.

4. Die MC4R-Aktivität verringerndes Mittel zur Verwendung nach Anspruch 3, wobei der Schritt des Überwachens einen Schritt des Überwachens des Gewichts des Individuums aufweist, und wobei die ausreichende Dosis bestimmt wird als die Mindestdosis, die eine Erhöhung des Körpergewichts des Individuums über einen festgesetzten Schwellenwert im Vergleich zu einem zweiten Individuum mit einem ähnlichen Symptom der ZNS-Erkrankung, dem aber die Zusammensetzung nicht verabreicht wird, veranlasst.

5. Die MC4R-Aktivität verringerndes Mittel zur Verwendung nach Anspruch 3, wobei das Individuum vor Verabreichung des Mittels eine verringerte Anzahl an dopaminergen Neuronen im Vergleich zu einem gesunden Individuum hat.

6. Die MC4R-Aktivtät verringerndes Mittel zur Verwendung nach Anspruch 1, wobei die Parkinson-Krankheit oder die Parkinson'schen Erkrankungen eine degenerative ZNS-Erkrankung ist, und wobei das Mittel für eine Zeitdauer verabreicht wird, bis das Individuum eine erwünschte Verringerung der Symptome zeigt, und wobei das Individuum für eine Dauer von mindestens zwei Wochen nach Beendigung der Verabreichung des Mittels eine fortdauernde Verringerung der Symptome zeigt.

7. Die MC4R-Aktivtät verringerndes Mittel zur Verwendung nach Anspruch 6, wobei die Behandlung die Proliferation adulter neuraler Stammzellen in der Seitenventrikelwand erhöht.

8. Die MC4R-Aktivtät verringerndes Mittel zur Verwendung nach Anspruch 6, wobei das Mittel verabreicht wird, um eine Gewebe-Konzentration von 0,1 nM bis 500 nM zu erreichen.

9. Die MC4R-Aktivität verringerndes Mittel zur Verwendung nach Anspruch 6, wobei das Mittel einem Individuum mit einer Verringerung an dopaminergen Neuronen im Gehirn verabreicht wird.

## Revendications

1. Agent réduisant l'activité de MC4R pour utilisation dans le traitement d'un trouble du système nerveux central (SNC) chez un sujet, lequel agent réduisant l'activité de MC4R est destiné à être administré au sujet en une quantité suffisante pour soulager un symptôme dudit trouble,
dans lequel le trouble du SNC est la maladie de Parkinson ou des troubles parkinsoniens ;
lequel agent est choisi dans l'ensemble constitué par HS014, HS028, le composé 10, le composé Pontillo14c, le composé Xi14a, le composé Xi14b, le composé Xi14c, le composé Xi14d, le composé Xi14e, le composé Xi14f, le composé Xi14g, le composé Xi14h, le composé Xi14i, le composé Xi14j, HS024, le composé 10d, le composé 18v, le composé 13b-2, le composé Tran12e, et leurs combinaisons.

2. Agent réduisant l'activité de MC4R pour utilisation selon la revendication 1, dans lequel est également administré au sujet
a) un ou plusieurs facteur(s) de croissance, ou
b) un ou plusieurs agent(s) choisi(s) dans l'ensemble constitué par les antidépresseurs, les agents anxiolytiques, les agents antipsychotiques, les agents antiépileptiques, les agents anti-maladie d'Alzheimer, les agents antiparkinsoniens, les inhibiteurs de MAO, les agents bloquant le captage de sérotonine, les agents bloquant le captage de noradrénaline, les agents bloquant le captage de dopamine, les agonistes de dopamine, la L-DOPA, les tranquillisants, les sédatifs, et le lithium.

3. Agent réduisant l'activité de MC4R pour utilisation selon la revendication 1, dans lequel ledit traitement comprend en outre
la surveillance d'un indice de néogenèse chez ledit sujet par un procédé non invasif pour déterminer si les doses sont suffisantes ; et
l'augmentation desdites doses si ledit indice de néogenèse est insuffisant, ou la diminution desdites doses si ledit indice de néogenèse est excessif.

4. Agent réduisant l'activité de MC4R pour utilisation selon la revendication 3, dans lequel ladite étape de surveillance comprend une étape de surveillance du poids dudit sujet, et dans lequel la dose suffisante est déterminée comme étant la dose minimale qui provoque une augmentation du poids corporel dudit sujet au-delà d'un seuil déterminé en comparaison avec un deuxième sujet présentant un symptôme similaire de trouble du SNC mais auquel ladite composition n'a pas été administrée.

5. Agent réduisant l'activité de MC4R pour utilisation selon la revendication 3, dans lequel ledit sujet comporte un nombre réduit de neurones dopaminergiques en comparaison avec un sujet en bonne santé avant administration dudit agent.

6. Agent réduisant l'activité de MC4R pour utilisation selon la revendication 1, dans lequel ladite maladie de Parkinson ou ledit trouble parkinsonien est un trouble du SNC dégénératif et ledit agent est administré jusqu'à ce que le sujet présente une réduction souhaitée des symptômes, et dans lequel ledit sujet présente une réduction continue des symptômes sur une période d'au moins deux semaines après que l'administration dudit agent a été stoppée.

7. Agent réduisant l'activité de MC4R pour utilisation selon la revendication 6, dans lequel ledit traitement augmente la prolifération de cellules souches neurales adultes dans la paroi ventriculaire latérale.

8. Agent réduisant l'activité de MC4R pour utilisation selon la revendication 6, lequel agent est administré pour atteindre une concentration tissulaire de 0,1 nM à 500 nM.

9. Agent réduisant l'activité de MC4R pour utilisation selon la revendication 6, lequel agent est administré à un sujet dont les neurones dopaminergiques sont réduits dans le cerveau.
